(19) **Europäisches Patentamt / European Patent Office / Office européen des brevets**

(11) **EP 4 679 432 A1**

(12) **EUROPEAN PATENT APPLICATION**
published in accordance with Art. 153(4) EPC

(43) Date of publication:
**14.01.2026 Bulletin 2026/03**

(21) Application number: **24767442.7**

(22) Date of filing: **07.03.2024**

(51) International Patent Classification (IPC):
*G16B 40/20* (2019.01)    *G16B 50/00* (2019.01)
*C12N 1/20* (2026.01)    *G06N 20/00* (2019.01)

(52) Cooperative Patent Classification (CPC):
**C12N 1/20; G06N 20/00; G16B 40/20; G16B 50/00**

(86) International application number:
**PCT/KR2024/002974**

(87) International publication number:
**WO 2024/186154 (12.09.2024 Gazette 2024/37)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **08.03.2023 KR 20230030822**
             **06.03.2024 KR 20240032154**

(71) Applicant: **Biomatz Co., Ltd.**
**Seoul 02792 (KR)**

(72) Inventors:
- **KIM, Yong Kyu**
**Gwacheon-si, Gyeonggi-do 13832 (KR)**

- **KIM, Sung Ki**
**Suwon-si, Gyeonggi-do 16493 (KR)**
- **LEE, Hyo Rim**
**Seoul 07297 (KR)**
- **NAM, Jeong Ah**
**Seoul 02748 (KR)**
- **KIM, Hyun Soo**
**Seoul 01671 (KR)**
- **KIM, Seo Jin**
**Seoul 04570 (KR)**
- **LEE, Ye Seul**
**Seoul 02492 (KR)**
- **LEE, Jae Kyoung**
**Namyangju-si, Gyeonggi-do 12010 (KR)**

(74) Representative: **Loyer & Abello**
**9, rue Anatole de la Forge**
**75017 Paris (FR)**

(54) **METHOD AND DEVICE FOR DETERMINING COMBINATION OF MICROBIAL STRAINS**

(57) Embodiments of the present application relate to methods of optimizing a combination of microbial strains and predicting the growth status of the corresponding strains. According to an embodiment of the present application for achieving the aforementioned task, a method of determining a combination of microbial strains may include: obtaining genome analysis information related to a target microorganism; obtaining first metabolic information related to each of a plurality of first microbial colonies including the target microorganism; estimating first growth index information related to each of the plurality of first microbial colonies by using the genome analysis information and the metabolic information as inputs of a first model; and determining a strain combination, based on at least one of the metabolic information and the growth index information.

FIG. 1

EP 4 679 432 A1

## Description

### Technical Field

The embodiments of the present application relate to methods of optimizing a combination of microbial strains and predicting the growth status of the corresponding strains. In particular, it may be applied to determine specific microbial strain combinations by using genome analysis, metabolic information, and growth index information, thereby optimizing productivity, efficiency, or production of specific metabolites.

### Background Art

The selection and combination of microbial strains plays a fundamental role in various fields such as biotechnology, pharmaceuticals, environmental engineering, and food engineering. These strains are used for a variety of purposes, including the production of specific compounds, the decomposition of harmful substances, and the expression of special biological effects. In particular, in industrial fermentation processes, an appropriate combination of microbial strains is essential to mass-produce specific substances, and in biological treatment processes, the selection of strains to effectively remove environmental pollutants is important.

Typically, the selection and combination of microbial strains have relied primarily on experimental methods. This includes evaluating the growth rates of various strains, the types and amounts of substances produced, their interactions with each other, and the like through large-scale culture experiments. However, this method not only requires a lot of time and labor, but also has limitations in finding the optimal combination among numerous possible combinations. Additionally, subtle differences in experimental conditions may have a large impact on the results, which may lead to issues with reproducibility.

The background art described above is technical information that the inventor possessed for deriving the present disclosure or obtained during the process of deriving the present disclosure, and may not necessarily be said to be technology disclosed to the general public prior to the application for the present disclosure.

### Disclosure of Invention

### Technical Problem

The various embodiments described herein are proposed to solve the aforementioned issues and provide a reinforcement learning method and device that considerrelationships such as cooperation and competition between objects in a complex cluster environment.

### Solution to Problem

According to an embodiment of the present application for achieving the aforementioned task, a method of determining a combination of microbial strains may include: obtaining genome analysis information related to a target microorganism; obtaining first metabolic information related to each of a plurality of first microbial colonies including the target microorganism; estimating first growth index information related to each of the plurality of first microbial colonies using the genome analysis information and the metabolic information as inputs of a first model; and determining a strain combination based on at least one or more of the metabolic information and the growth index information.

Each of the plurality of first microbial colonies may consist of an arbitrary multi strain combining sequence data of one or more single strains and the genome data of the target microorganism.

The genome analysis information may include species composition data, predicted gene data, and metabolite data related to the target microorganism.

The species composition data includes phylogenetic information of the target microorganism, and specifically, may include at least one of species classification information, strain classification information, phylogenetic tree information, and phylogenetic distance information, etc., of the target microorganism.

The predicted gene data includes information on all genes that may be derived from the whole genome sequence of the target microorganism, and may include a list of all encoded gene types and/or functional annotation data of the genes. In addition, the predicted gene data may include atleast one or more of the following: metabolism-related genetic information, antibiotic-related genetic information, toxin genetic information, optimal medium composition information, and biosynthetic gene group information of the target microorganism.

The metabolite data may include information on metabolites identified by estimating biochemical pathways and metabolic pathways derived based on information on all genes that may be identified from the full genome sequence of the target microorganism and information on proteins (enzymes) encoded by the genes.

The metabolic information may include the results of analyzing metabolic interactions between microorganisms in two or more microbial colonies. The results of the metabolic interaction analysis may be analyzed by considering the results of predicting metabolic resource overlap between microorganisms, metabolic interaction potential, metabolic mismatch, and/or minimum nutrient diversity for growth, etc., and specifically, may include at least one or more of metabolic resource overlap (MRO) and metabolic interaction potential (MIP).

The metabolic resource overlap (MRO) is a value calculated by measuring the similarity of metabolites required by each microorganism within a microbial community when they exist independently, and is an indicator of the degree of competition between microorganisms for a given nutrient in the community, and may be calculated using the following Equation 1.

[Equation 1]

$$MRO = \frac{n \sum_{i,j|i\neq j}|M_i \cap M_j|}{C(n,2)\sum_{i\neq 0}^{n}|M_i|}$$

($M_i$: the minimum number of nutrients required for the growth of each microorganism i in a community consisting of n species)

The metabolic interaction potential (MIP) is a value representing the maximum number of metabolites that may be exchanged between microorganisms existing in a microbial community, and is an indicator of metabolic dependence between microorganisms constituting the community, and may be calculated using the following Equation 2.

[Equation 2]

$$MIP = M - I$$

(M: the minimum number of metabolites required when each community constituting microorganism exists independently; I: the minimum number of metabolites required for community growth when metabolite exchange is allowed within the community)

The MRO and MIP may be calculated using the SMETANA tool, and detailed descriptions of the algorithm of the SMETANA analysis tool are described in the publication, "Metabolic dependencies drive species co-occurrence in diverse microbial communities (PNAS May 19, 2015 112 (20) 6449-6454)."

The growth index information refers to information on an indicator that may confirm or evaluate the growth, growth and/or proliferation level of a microbial colony (multi strain group) including one microorganism or two or more microorganisms. In an embodiment, the growth index information may include at least one or more selected from the group consisting of optical density (OD) information measured for microbial growth, etc., using spectrophotometry, time to reach the maximum OD value (Time to maxOD), growth/growth rate, and growth/-growth rate by section (lag phase, exponential growth phase, stationary phase, death phase) in the microbial growth phase, and may include, without limitation, any information that may confirm whether or not the microorganism is growing and the growth level. In addition, the growth index information may include a comparison value [△(single/community)] between the growth index information of an arbitrary single microorganism and the growth index information of a multi strain group including the arbitrary single microorganism.

The first metabolic information may include at least one or more of metabolic resource overlap (MRO) data and metabolic interaction potential (MIP) data.

The first model may be a regression model using the genome analysis information and the first metabolic information as features.

The method may further include obtaining second metabolic information and second growth index information related to each of a plurality of second microbial colonies stored in a database; and determining the strain combination by using the first metabolic information, the second metabolic information, the first growth index information, and the second growth index information as inputs of a second model.

The second model may include a latent factor collaborative filtering algorithm model, and determining the strain combination may include: using the target microorganism and a single strain as a user; using third metabolic information and third growth index information of data concatenated with the first metabolic information, the second metabolic information, the first growth index information, and the second growth index information as items; and determining a microbial strain combination for at least one of the third metabolic information and the third growth index information.

Determining the strain combination may further include re-determining the determined strain combination by determining the weight of each of the estimated third metabolic information and the estimated third growth index information.

The second model may include a transformer model, and determining the strain combination may include determining the strain combination based on the second model trained on the relationship between the target microorganism and one or more single strains, based on the first metabolic information, the second metabolic information, the first growth index information, and the second growth index information.

According to an embodiment of the present application for achieving the aforementioned task, a method of generating a model for estimating growth index information includes: obtaining genome analysis information on a plurality of microorganisms; obtaining metabolic information and growth index information on each of a plurality of microbial colonies including each of the plurality of microorganisms; generating a data set having the genome analysis information and the metabolic information as features and the growth index information as a label; and generating a model for estimating growth index information on each of the plurality of microbial colonies included based on the data set.

According to an embodiment of the present application for achieving the aforementioned task, a computer device includes a processor, wherein the processor obtains genome analysis information related to a target microorganism, obtains first metabolic information related to each of a plurality of first microbial colonies including the target microorganism, estimates first growth index information related to each of the plurality of first microbial colonies using the genome analysis information and the metabolic information as inputs of a first model, and determines a strain combination based on the growth index information.

Each of the plurality of first microbial colonies may consist of an arbitrary multi strain combining sequence data of one or more single strains and the genome data of the target microorganism.

The genome analysis information may include species composition data, predicted gene data, and metabolite data related to the target microorganism.

The first metabolic information may include at least one of metabolic resource overlap (MRO) data and metabolic interaction potential (MIP) data.

The first model may be a regression model using the genome analysis information and the first metabolic information as features.

The processor may obtain second metabolic information and second growth index information related to each of a plurality of second microbial coloniesstored in a database, and determine the strain combination by using the first metabolic information, the second metabolic information, the first growth index information, and the second growth index information as inputs of a second model.

The second model includes a latent factor collaborative filtering algorithm model, and the processor uses the target microorganism and the single strain as a user, and uses third metabolic information and third growth index information of data concatenated with the first metabolic information, the second metabolic information, the first growth index information, and the second growth index information as items, and may determine a microbial strain combination for at least one of the third metabolic information and the third growth index information.

The processor may re-determine by determining the weights of each of the estimated third metabolic information and the third growth index information.

The second model includes a transformer model, and the processor may learn a relationship between the target microorganism and a single strain based on the first metabolic information, the second metabolic information, the first growth index information, and the second growth index information ,and determine a strain combination based on the relationship between the target microorganism and the single strain.

The combination of microbial strains determined or derived using the method, computer device and/or processor of the present disclosure may be a combination of strains for improving the functionality of target microorganisms. Improving the functionality may be to improve the growth, activity (including physiological activity or pharmacological activity), stability and/or intestinal colonization ability of the target microorganism.

**Advantageous Effects of Invention**

According to an embodiment of the present disclosure, various object types and their characteristics within a cluster may be effectively identified and reflected, so that each object may determine the optimal action suitable for its role and situation.

The effects of the present disclosure are not limited to the effects mentioned above.

**Brief Description of Drawings**

FIG. 1 is a diagram schematically illustrating a process of determining a strain combination of a computer device according to an embodiment of the present disclosure.

FIG. 2 is a diagram schematically illustrating a process of determining a strain combination of a computer device according to an embodiment of the present disclosure.

FIG. 3 is a flowchart illustrating an operation of estimating growth index information of a computer device according to an embodiment of the present disclosure.

FIG. 4 is a diagram schematically illustrating a process of obtaining genome analysis information of a computer device according to an embodiment of the present disclosure.

FIG. 5 is a diagram schematically illustrating a process of obtaining metabolic information of a computer device according to an embodiment of the present disclosure.

FIG. 6 is a conceptual diagram schematically illustrating a process of obtaining metabolic information by analyzing, as a single strain, a multi strain of a computer device according to an embodiment of the present disclosure.

FIG. 7 is a diagram schematically illustrating a process of estimating growth index information of a computer device according to an embodiment of the present disclosure.

FIG. 8 is a flowchart illustrating an operation of determining a strain combination of a computer device according to an embodiment of the present disclosure.

FIG. 9 is a diagram schematically illustrating a process of determining a strain combination on the basis of collaborative filtering of a computer device according to an embodiment of the present disclosure.

FIG. 10 is a diagram schematically illustrating a process of determining a strain combination on the basis of a ranking model of a computer device according to an embodiment of the present disclosure.

FIG. 11 is a block diagram schematically illustrating the configuration of a computer device according to an embodiment.

**Mode for the Invention**

The terms used in the present disclosure are only used to describe specific embodiments and may not be intended to limit the scope of other embodiments. A singular expression may include a plural expression unless the context clearly indicates otherwise. Terms used herein, including technical or scientific terms, may have the same meaning as commonly understood by a person of ordinary skill in the art described in the present disclosure. Among the terms used in the present disclosure, terms defined in general dictionaries may be interpreted as having the same or similar meaning as the meaning they have in the context of the related technology, and shall not be interpreted in an ideal or overly formal meaning unless explicitly defined in the present disclosure. In some cases, even if a term is defined in the present disclosure, it may not be interpreted to exclude embodiments of the present disclosure.

Below, various embodiments are described in detail with reference to the attached drawings so that a person of ordinary skill in the art may easily implement the present disclosure. However, the technical idea of the present disclosure may be implemented in various forms and is not limited to the embodiments described in the present application. In describing the embodiments disclosed in the present application, if it is determined that a specific description of a related known technology may obscure the gist of the technical idea of the present disclosure, a specific description of the known technology is omitted. Identical or similar components are assigned the same reference number and duplicate descriptions thereof are omitted.

In this case, the term '~unit' used in the present embodiment refers to a componentthat performs a specific function performed by software or hardware such as an FPGA (Field Programmable Gate Array) or ASIC (Application Specific Integrated Circuit). However, '~unit' is not limited to being performed by software or hardware. The '~unit' may exist in the form of data stored in an addressable storage medium, or may be implemented by instructions so that one or more processors are configured to execute a specific function.

Software may include a computer program, code, instruction, or a combination of one or more of these, which may configure a processing device to perform a desired operation or may independently or collectively instruct the processing device. The software and/or data may be permanently or temporarily embodied in anytype of machine, component, physical device, virtual equipment, computer storage media or device, or transmitted signal waves, for interpretation by a processing device or for providing instructions or data to a processing device. The software may be distributed across computer systems connected via a network, and may be stored or executed in a distributed manner. The software and data may be stored on one or more computer-readable recording media. The software may be read into main memory from another computer-readable medium, such as a data storage device, or from anotherdevice via a communications interface. Software instructions stored in main memory may cause the processor to perform processes or steps, which will be described in detail below. Alternatively, hardwired circuitry may be used instead of, or in combination with, software instructions to execute processes consistent with the principles of the present disclosure. Therefore, embodiments consistent with the principles of the present disclosure are not limited to any specific combination of hardware circuits and software.

The terminology used in the present application is used only to describe particular embodiments and is not intended to limit the present disclosure. Singular expressions include plural expressions unless the context clearly indicates otherwise. In the present application, it should be understood that terms such as "includes" or "has," etc., are intended to specify the presence of a feature, number, step, operation, component, part or combination thereof described in the disclosure, but do not exclude in advance the possibility of the presence or addition of one or more other features, numbers, steps, operations, components, parts or combinations thereof. The terms first, second, etc. may be used to describe various components, but the components should not be limited by these terms. The terms are used solely for the objective of distinguishing one component from another.

The 'model' referred to in the present disclosure may include any form of algorithm or methodology used to learn or understand a specific pattern or structure from data. Models may include machine learning models such as regression models, decision trees, random forests, support vector machines, K-nearest neighbors, naive bayes, and clustering algorithms, etc., as well as deep learning models such as neural networks, convolutional neural networks, recurrent neural networks, transformer-based neural networks, generative adversarial networks (GANs), and autoencoders,etc. A 'model' may refer to a set of trained parameters or weights that are used to predict or classify outputs for a certain input, and the model may be trained using methods such as supervised learning, unsupervised learning, semi-supervised learning,or reinforcement learning, etc. Additionally, it may include various learning methods and structures, such as single models, ensemble models, multi-modal models, and models by transfer learning. These models may be pre-trained on a separate computer device from the one that predicts output for input, and may be used on another computer device.

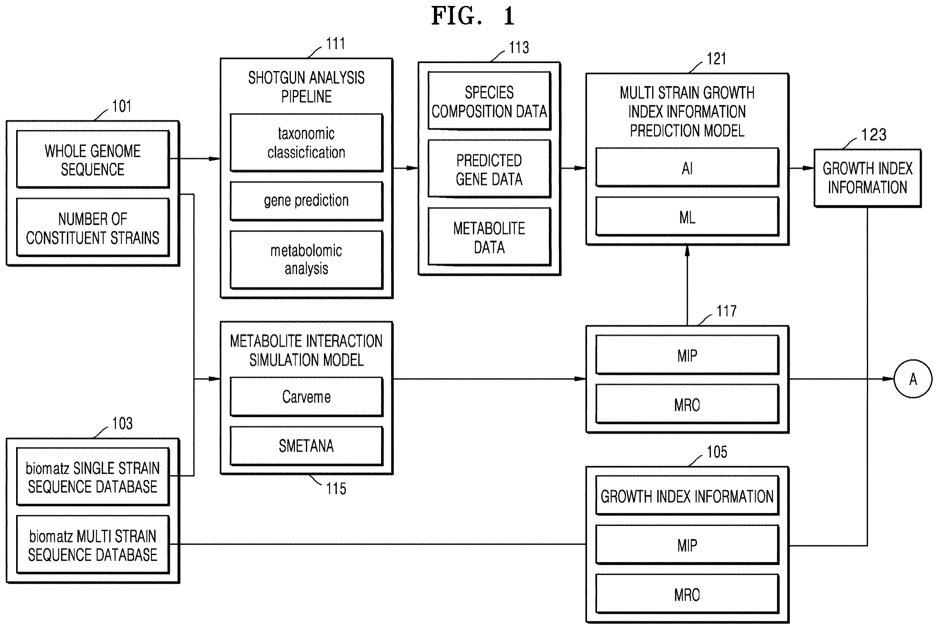

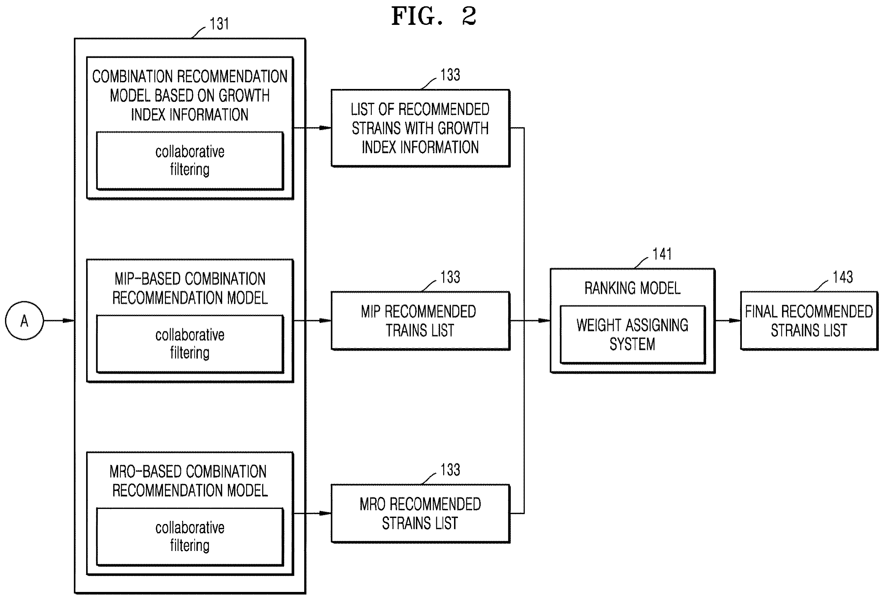

FIG. 1 and FIG. 2 are diagrams schematically illustrating a process of determining a strain combination of the computer device according to an embodiment of the present disclosure.

Referring to FIG. 1 and FIG. 2, a computer device may obtain user input 101 from a user. Such user input 101 may include genome sequence data of the target microorganism and the number of constituent strains. These target genome sequence data include genetic information of microorganisms, and the strain number may indicate the number of constituent strains for strain combination. That is, the user may input the genome sequence data of the target microorganism to form a colony and the number of constituent strains to be included in the colony.

The computer device may obtain single strain sequence data and multi strain sequence data from the database 103. These single strain sequence data may point to the genome sequence data of each single strain. Additionally, multi strain sequence data may indicate one or more co-occurring groups (forms) in which two or more microbial species are mixed, and genome sequence data related to microorganisms belonging to each of the co-occurring groups. Specifically, the above co-occurring group may include a collection of microorganisms that are actually coexisting or forming colonies, isolated or identified from various environments including the intestines and/or feces, etc. A computer device may obtain genome analysis information 113 related to a target microorganism based on user input 101, and as an example, may obtain it using a shotgun analysis pipeline 111. Specifically, the computer device may extract DNA of a target microorganism and, if necessary, amplify the DNA. Computer devices may obtain the sequence by randomly breaking the DNA into thousands or millions of smaller pieces. The computer device may use the obtained sequence data to reconstruct a genome sequence, predict the location of genes, the list of all encoded gene types, and/or functional annotations of the genes, etc in the assembled genome. Genome analysis information 113 may include species composition data, predicted gene data, and metabolite data related to the target microorganism.

Additionally, the computer device may obtain metabolic information 117 based on a database 103, user input 101, and a metabolite interaction simulation model 115. Such metabolic information may include at least one of Metabolic Resource Overlap (MRO) data and Metabolic Interaction Potential (MIP) data. For example, as an example, the metabolite interaction simulation model 115 may include at least one of CarveMe, which automatically generates genome-based metabolic models, and SMETANA, which predicts metabolic interactions within a microbial community. The computer device may calculate MRO and MIP values by using the genome sequence data of the target microorganism and the genome sequence data of the strain included in the database in the metabolite interaction simulation model 115.

The computer device may estimate maximum optical density (maxOD) 123 as an example of growth index information of a colony including a target microorganism by inputting genome analysis information and metabolic information into a first model 121, which is a regression model that uses genome analysis information and metabolic information as features and growth index information as labels.

The computer device may input metabolic information and growth index information 105 of multi strains stored in a database and metabolic information and growth index information of a colony including microorganisms into a second model 131 to determine a first strain combination 133.

Furthermore, the computer device may re-determine the first strain combination 133 as a second strain combination 143 by inputting the first strain combination 133 back into a ranking model 141 to determine the weights.

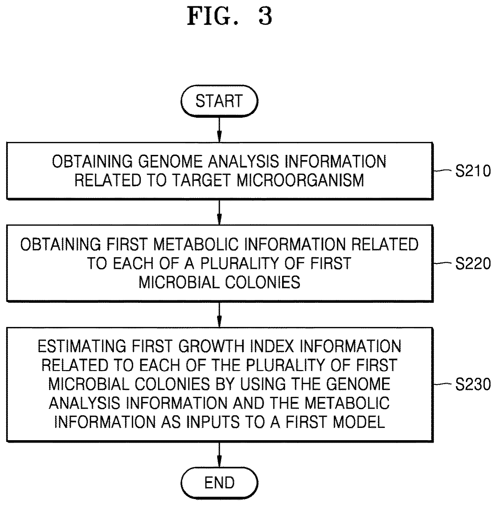

FIG. 3 is a flowchart illustrating an operation of estimating growth index information of the computer device according to an embodiment of the present disclosure.

Referring to FIG. 3, the computer device may obtain genome analysis information on a target microorganism at step S210.

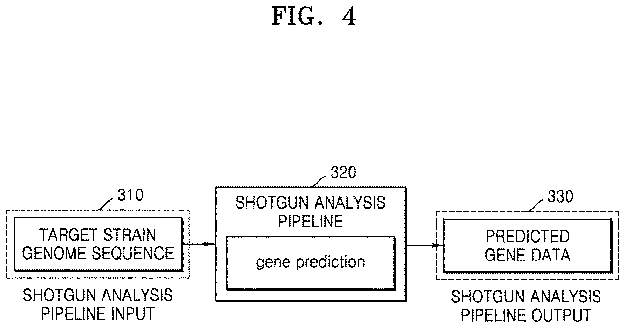

For example, a computer device may obtain genome analysis information on a target microorganism based on a shotgun sequencing pipeline 320 as illustrated in FIG. 4. The computer device may extract DNA using the genome sequence data of the target microorganism 310 and divide it into small pieces. For example, a computer device may use a variety of chemical and physical methods to destroy the cell wall of a target microorganism, isolate the DNA inside the cell, and break down the DNA into smaller pieces by physical or enzymatic methods. Computer devices may determine the nucleotide sequence of broken down DNA fragments through DNA sequencing. For example, a computer device may sequence DNA fragments by identifying their nucleotide sequences using Illumina sequencing or Nanopore sequencing. Computer devices may reconstruct the original genome sequence by reassembling short fragments of DNA sequence. Computer devices may be used to match overlapping sequence fragments, through this, generating contigs, the longest possible continuous DNA sequence. Computer devices may predict the location and function of genes from reconstructed genome sequences. Such predicted gene data 330 may include at least one or more of species composition data, predicted gene data, and metabolite data related to the target microorganism as genome analysis information related to the target microorganism.

A computer device according to an embodiment may obtain first metabolic information related to each of a plurality of first microbial colonies including a target microorganism, at step S220. Each of these plurality of first microbial colonies may consist of an arbitrary multi strain combining sequence data of a single strain and the genome data of the target microorganism. The first metabolic information may include at least one of metabolic resource overlap (MRO) data and metabolic interaction potential (MIP) data.

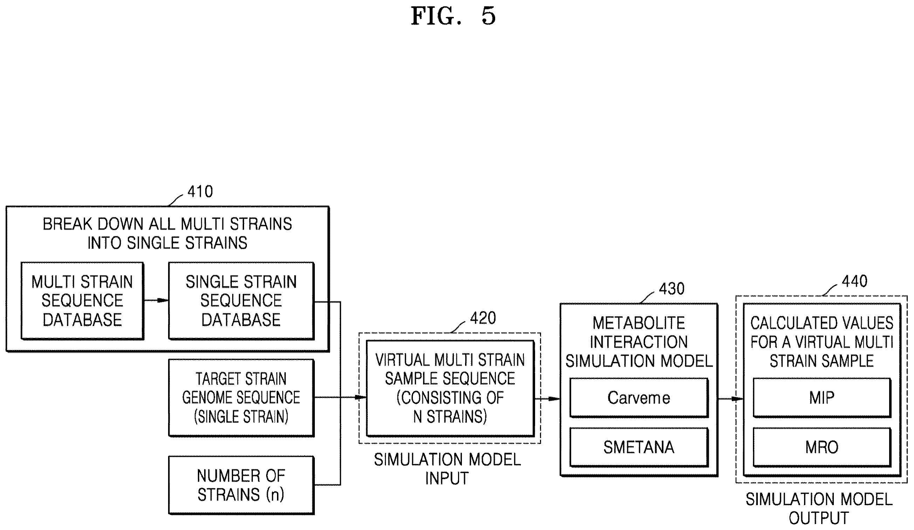

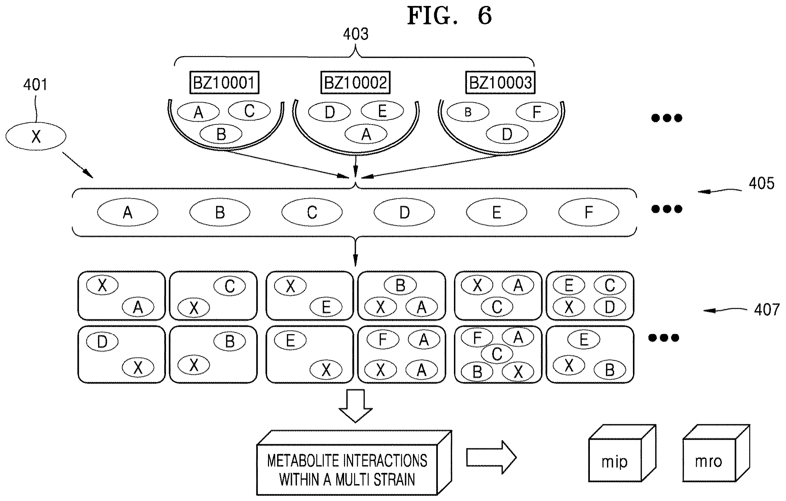

For example, a computer device may generate a multi strain sample 420 using the genome sequence data of the target microorganism, the number of constituent strains, and the single strain sequence data 410 as illustrated in FIG. 5. A multi strain sample 420 may be an arbitrary multi strain that combines sequence data of a single strain and the genome data of the target microorganism, and may indicate a plurality of first microbial colonies including the target microorganism. These multi strain samples 420 are described in detail by FIG. 6.

The computer device may obtain first metabolic information 440 using a multi strain sample 420 and a metabolite interaction simulation 430. A computer device may reconstruct a metabolic network from a genome sequence using CarveMe based on sequence data of a multi strain sample 420. Alternatively, a computer device may simulate metabolite exchange and competition from genome sequences using SMETANA to analyze synergistic effect and competitive relationships between strains.

FIG. 6 is a conceptual diagram schematically illustrating a process of obtaining metabolic information by analyzing a multi strain 403 as a single strain 405 and then reconstructing it as a multi strain 407 reconstructed through simulation including a target microorganism 401. Computer devices may analyze microbial communities using approaches from bioinformatics and systems biology. The multi strain 403 is data included in a database, etc., and a computer device may individually analyze each microbial strain that constitutes the multi strain 403. For example, the computer device may analyze the genome information, metabolic pathways, functional properties, etc. of each microorganism based on at least one method of high-performance DNA sequencing, genome interpretation, and metagenomic analysis. The computer device may construct a new multi strain including a target microorganism 401 based on data obtained through a single strain 405. Thereafter, the computer device may obtain metabolic information by applying a metabolite interaction simulation to a new multi strain including the target microorganism 401.

The computer device according to an embodiment may estimate the first growth index information related to each of a plurality of first microbial colonies using genome analysis information and metabolic information as inputs of the first model at step S230.

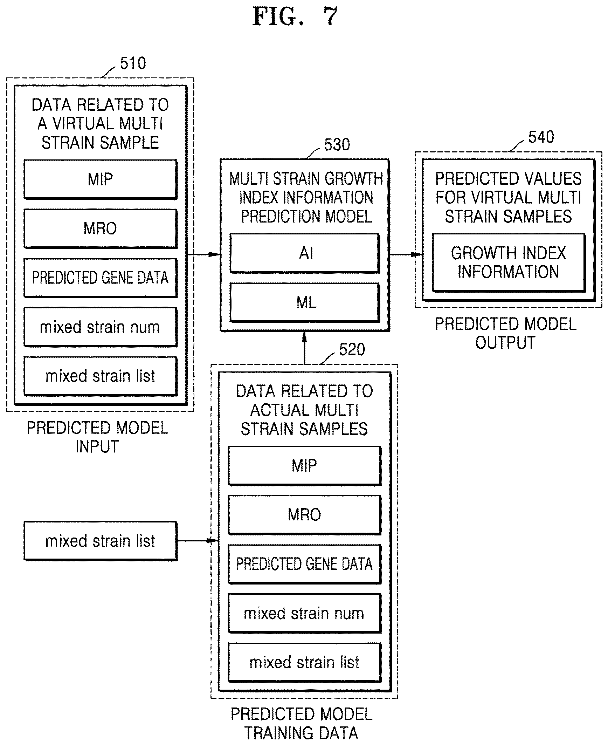

For example, the computer device may train a first model 530 that estimates growth index information using multi strain sample data 520 stored in a database as illustrated in FIG. 7, and may estimate growth index information 540 of each of a plurality of first microbial colonies 510 by inputting genome analysis information and metabolic information of each of a plurality of first microbial colonies 510 including target microorganisms reconstructed through simulation. That is, the first model is a model for estimating growth index information, and may be a regression model trained using a data set that uses genome analysis information related to a plurality of microorganisms, metabolic information and growth index information related to each of a plurality of microbial colonies including each of the plurality of microorganisms as features, and the growth index information as a label. As an example, the first model may be a regression model trained using, as a dataset, at least one or more of second metabolic information, second growth index information for each of a plurality of second microbial colonies stored in a database, and genome analysis information on single strains constituting each of the plurality of second microbial colonies.

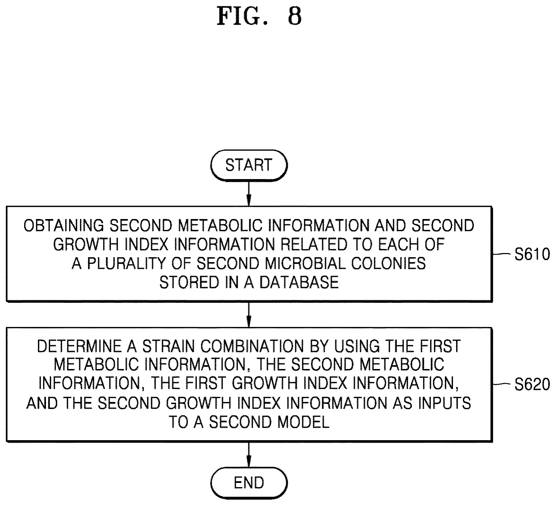

FIG. 8 is a flowchart illustrating an operation of determining a strain combination of the computer device according to an embodiment of the present disclosure.

Referring to FIG. 8, the computer device may obtain second metabolic information and second growth index information related to each of a plurality of second microbial colonies stored in the database at step S610.

The computer device according to an embodiment may determine a combination of strains including a target microorganism at step S620 by using a) first metabolic information related to each of a plurality of first microbial colonies including a target microorganism and first growth index information related to each of the plurality of first microbial colonies, and b) second metabolic information and second growth index information related to each of a plurality of second microbial colonies stored in a database.

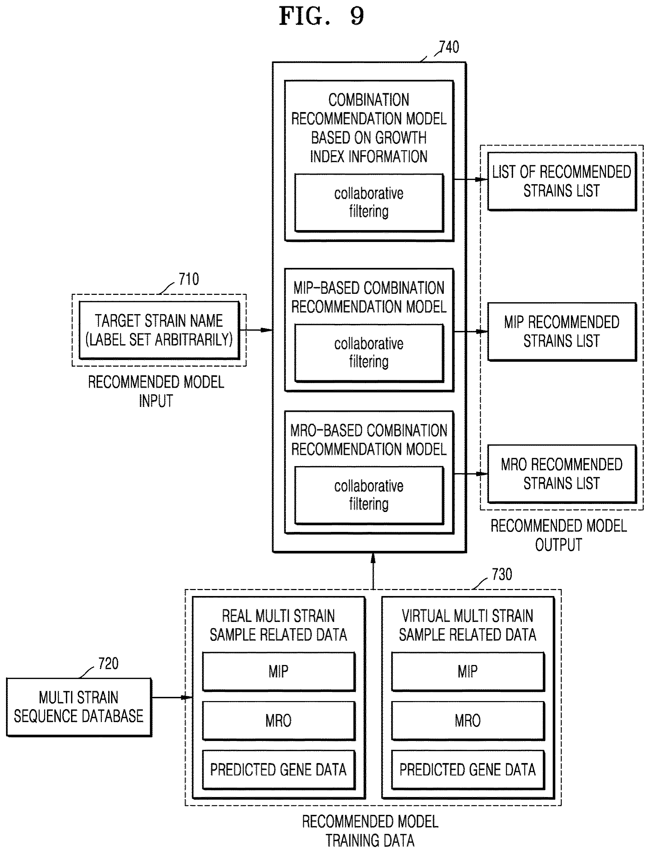

For example, the computer device may determine a strain combination based on a collaborative filtering algorithm model as illustrated in FIG. 9. As illustrated in FIG. 9, the user-item evaluation matrix of the latent factor model may be trained using the metabolic information and growth index information of the multi strain 720 stored in the database and the metabolic information and growth index information of the strain sample 730 including the target microorganism. The loss function of this latent factor model may be expressed as shown in Equation 3.

[Equation 3]

$$min\sum\left(r_{(u,i)} - p_u q_i^T\right)^2 + \lambda\left(\|q_i\|^2 + \|p_u\|^2\right)$$

r(u,i) may indicate an actual rating value between user u and item i, which is the value of row u, column i of the R matrix, $p_u$ may indicate the vector of the user u row of the P matrix when the actual R matrix is factorized into the P matrix and Q matrix including latent factors, $q_i^T$ may indicate the transpose vector of the item i row of the Q matrix when the actual R matrix is factorized into the P matrix and Q matrix including latent factors, and $\lambda$ may indicate a regularization parameter multiplied by the regularization term.

The predicted R matrix value is calculated by taking the inner product of the P matrix and the Q matrix, and the principle of the latent factor collaborative filtering model is to repeatedly optimize the cost function so that it may have the minimum error with the actual R matrix value. Additionally, a regularization term may be added to prevent overfitting of the data.

Specifically, a latent factor model may be trained by using the target microorganism and single strain as user u, and the third metabolic information and the third growth index information of the data concatenated with the first metabolic information, the second metabolic information, the first growth index information, and the second growth index information as item i. In addition, a latent factor model may be trained by using the target microorganism and single strain as item i, and the third metabolic information and the third growth index information of the data concatenated with the first metabolic information, the second metabolic information, the first growth index information, and the second growth index information as user u.

Latent factor vectors represent hidden characteristics of users (target microorganism) or items (metabolites), which are not directly observed but may be inferred through the model. The computer device obtains first metabolic information and first growth index information of a plurality of first microbial colonies including target microorganisms. In addition, the second metabolic information and second growth index information of other microbial colonies stored in the database are obtained, and the metabolic information and growth index information may be used as inputs to the model.

Furthermore, the computer device may also use GridSearchCV from the Surprise library to search for optimal model hyperparameters. In this case, the hyperparameters being optimized may be n_factors (the number of latent factor dimensions), lr_all (learning rate), and reg_all (regularization parameter) of the latent factor model.

A computer device according to another embodiment may determine a strain combination based on a collaborative filtering algorithm model at step S620, and may determine the strain combination by adjusting the weights of growth index information and metabolic information according to the purpose. For example, a computer device may determine a strain combination using a ranking model that adjusts the weights of growth index information, MIP, and MRO values, assigning a higher weight to the growth index information value if rapid growth is desired, assigning a higher weight to the MIP value if the purpose is cooperative colonization, and assigning a higher weight to the MRO value if the purpose is to suppress a specific target strain.

A computer device according to another embodiment may determine a strain combination based on a second model trained on the relationship between a target microorganism and a single strain based on the first metabolic information, the second metabolic information, the first growth index information, and the second growth index information at step S620. For example, a computer device may determine strain combinations based on a transformer model. The computer device may use an attention mechanism to assign weights to the relationships between data points according to their importance, and may be trained, based on metabolic information and growth index information, whether two microbial species are in a competitive relationship that inhibits each other's growth, or whether they are in a symbiotic relationship that exhibits a synergistic effect through mutually beneficial interactions, etc.

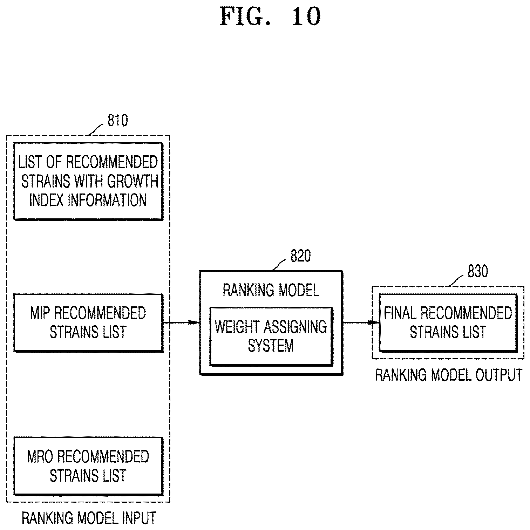

FIG. 10 is a diagram schematically illustrating a process of determining a strain combination on the basis of a ranking model of the computer device according to an embodiment of the present disclosure.

Referring to FIG. 10, the computer device may output a list of recommended strains with growth index information values as items, a list of recommended strains with metabolic interaction potential (MIP) values as items, and a list of recommended strains with metabolic resource overlap (MRO) values as items based on a collaborative filtering algorithm model of strains. The computer device may use these lists as inputs 810 of a ranking model 820 to determine a final recommended strain list 830 as a combination of strains. The ranking model 820 may assign weights to each input data set according to the user's purpose. After the weights are applied, the computer device may calculate a comprehensive score for each strain according to the ranking model to select a combination of strains with high scores.

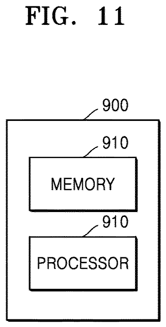

FIG. 11 is a block diagram schematically illustrating the configuration of the computer device according to an embodiment.

The computer device is illustrated as being configured by a memory 910 and a processor 920, but is not necessarily limited thereto. The memory 910 and the processor 920 may each exist as a physically independent component.

The memory 910 may store various data for the overall operation of the computer device, such as a program for processing or controlling the processor 920 in the computer device.

A memory 910 according to an embodiment may store multi strain data, single strain data, large-scale data sets, algorithms, analysis models, etc., and the memory 910 may store a plurality of application programs being run, data for the operation of a computer device, and commands. The memory 910 may be implemented as an internal memory such as a ROM, RAM, or solid state drive (SSD), etc., included in the processor 920, or may be implemented as a separate memory from the processor 920. A memory 910 according to an embodiment may store models and training data.

The processor 920 may be configured to control the overall computer device. For example, the processor 920 may control a computer device to perform operations according to an embodiment of the present disclosure.

A processor 920 according to an embodiment may obtain genome analysis information related to a target microorganism, obtain first metabolic information related to each of a plurality of first microbial colonies including the target microorganism, estimate first growth index information related to each of the plurality of first microbial colonies using the genome analysis information and the metabolic information as inputs of a first model, and determine a strain combination based on the growth index information.

The processor 920 according to an embodiment may obtain second metabolic information and second growth index information related to each of a plurality of second microbial colonies stored in a database, and determine a strain combination by using the first metabolic information, the second metabolic information, the first growth index information, and the second growth index information as inputs to a second model.

The processor 920 according to an embodiment receives genome sequence data of the target microorganism for forming a colony and the number of constituent strains to be included in the colony, uses the target microorganism and a single strain as a user, and uses third metabolic information and third growth index information of data concatenated with first metabolic information, second metabolic information, first growth index information, and second growth index information as items, and may determine a microbial strain combination for at least one of the third metabolic information and the third growth index information. In this case, each combination of microbial strains may be a combination composed of as many strains as the number of constituent strains input by the user.

The processor 920 according to an embodiment may re-determine the determined strain combination by determining the weight of each of the estimated third metabolic information and the estimated third growth index information.

The processor 920 according to an embodiment may learn a relationship between the target microorganism and a single strain based on the first metabolic information, the second metabolic information, the first growth index information, and the second growth index information, and determine a strain combination based on the relationship between the target microorganism and the single strain.

Specifically, the processor 920 may control the operation of the computer device using various programs stored in the memory 910 of the computer device. The processor 920 may include a CPU, RAM, ROM, a system bus, etc. The processor 920 may be implemented as a single CPU or multiple CPUs (or DSP, SoC). As an example, the processor 920 may be implemented as a digital signal processor (DSP), a microprocessor, or a time controller(TCON) that processes digital signals. However, it is not limited thereto, and may include one or more of a central processing unit (CPU), a micro controller unit (MCU), a micro processing unit (MPU), a controller, an application processor (AP), a communication processor (CP), or an ARM processor, or may be defined by such terms. Additionally, the processor 920 may be implemented as a System on Chip (SoC), large scale integration (LSI) with a built-in processing algorithm, or may be implemented in the form of a Field Programmable Gate Array (FPGA). To efficiently execute deep learning and data processing algorithms, the processor 920 may also include components specialized for high-performance computing. These components may include a Neural Processing Unit (NPU), a Graphics Processing Unit (GPU), a Tensor Processing Unit (TPU), etc.

As described above, although the embodiments have been explained with reference to limited embodiments and drawings, it will be understood by a person of ordinary skill in the art that various modifications and variations may be made based on the above disclosure. For example, suitable results may be achieved even if the described techniques are performed in an order different from the described methods, and/or components of the described systems, structures, devices, circuits, etc. are combined or arranged in a different manner than described, or are replaced or substituted by other components or equivalents.

Therefore, other implementations, other embodiments, and equivalents to the claims are also included in the scope of the claims described below.

[Description of Reference Numerals]

101: User Input 103: Database
105: Metabolic Information and Growth Index Information 111: Shotgun Analysis Pipeline
113: Genome Analysis Information 115: Metabolite Interaction Simulation Model
117: Metabolic Information 121: First Model, Which is a Regression Model
123: Growth Index Information 131: Second Model
133: First Strain Combination 141: Ranking Model
143: Second Strain Combination 310: Genome Sequence Data of Target Microorganism
320: Shotgun Sequencing Pipeline 330: Predicted Gene Data
401: Target Microorganism 403: Multi Strain
405: Single Strain 407: Multi Strain Reconstructed Through Simulation
410: Single Strain Sequence Data 420: Multi Strain Sample
430: Metabolite Interaction Simulation 440: First Metabolic Information
510: Plurality First of Microbial Colonies 520: Multi Strain Sample Data
530: First Model 540: Growth Index Information
720: Multi strain 730: Strain Sample
810: Input 820: Ranking Model
830: Final Recommended Strain List 910: Memory
920: Processor

**Claims**

1. A method of determining a microbial strain combination, the method comprising:

obtaining genome analysis information related to a target microorganism; obtaining first metabolic information related to each of a plurality of first microbial colonies comprising the target microorganism;

estimating first growth index information related to each of the plurality of first microbial colonies by using the genome analysis information and the metabolic information as inputs to a first model; and

determining a strain combination, based on at least one of the metabolic information and the growth index information.

2. The method of claim 1,
   wherein each of the plurality of first microbial colonies is composed of an arbitrary multi strain formed by combining sequence data of one or more single strains and genome data of the target microorganism.

3. The method of claim 1,
   wherein the genome analysis information comprises species composition data, predicted gene data and metabolite data, which are related to the target microorganism.

4. The method of claim 1,

   wherein the first metabolic information comprises

   at least one of metabolic resource overlap (MRO) data and metabolic interaction potential (MIP) data.

5. The method of claim 1,
   wherein the first model is a regression model trained using, as a dataset, second metabolic information and second growth index information for each of a plurality of second microbial colonies stored in a database, and genome analysis information on single strains constituting each of the plurality of second microbial colonies.

6. The method of claim 1, further comprising:

   obtaining second metabolic information and second growth index information related to each of a plurality of second microbial colonies stored in a database; and

   determining the strain combination by using the first metabolic information, the second metabolic information, the first growth index information, and the second growth index information as inputs to a second model.

7. The method of claim 6,

wherein the second model comprises a latent factor collaborative filtering algorithm model, and

the determining of the strain combination comprises:

   using the target microorganism and a single strain as a user;

   using, as items, third metabolic information and third growth index information of data concatenating the first metabolic information, the second metabolic information, the first growth index information, and the second growth index information; and

   determining a microbial strain combination for at least one or more of the third metabolic information and the third growth index information.

8. The method of claim 7,
   wherein the determining of the strain combination further comprises re-determining the strain combination by determining weights for each of the estimated third metabolic information and the estimated third growth index information.

9. The method of claim 6,
   wherein the second model comprises a transformer model, and the determining of the strain combination comprises determining the strain combination, based on the second model trained on a relationship between the target microorganism and one or more single strains, based on the first metabolic information, the second metabolic information, the first growth index information, and the second growth index information.

10. A method of generating a model for estimating growth index information, the method comprising:

   obtaining genome analysis information related to a plurality of microorganisms;

   obtaining metabolic information and growth index information related to each of a plurality of microbial colonies comprising each of the plurality of microorganisms;

   generating a dataset by using the genome analysis information and the metabolic information as features, and the growth index information as labels; and

   , based on the dataset, generating a model for estimating growth index information related to each of the plurality of microbial colonies.

11. The method of claim 10,

   wherein the metabolic information comprises
   at least one of metabolic resource overlap (MRO) data and metabolic interaction potential (MIP) data.

12. A computer device comprising:

a processor,
wherein the processor
obtains genome analysis information related to a target microorganism,
obtains first metabolic information related to each of a plurality of first microbial colonies comprising the target microorganism, estimates first growth index information related to each of the plurality of first microbial colonies by using the genome analysis information and the metabolic information as inputs to a first model, and, based on at least one of the metabolic information and the growth index information, determines a strain combination.

13. The computer device of claim 12,
wherein each of the plurality of first microbial colonies consists of an arbitrary multi strain formed by combining sequence data of a single strain and genome data of the target microorganism.

14. The computer device of claim 12,
wherein the genome analysis information comprises species composition data, predicted gene data and metabolite data, which are related to the target microorganism.

15. The computer device of claim 12,
wherein the first metabolic information comprises at least one of metabolic resource overlap (MRO) data and metabolic interaction potential (MIP) data.

16. The computer device of claim 12,
wherein the first model is trained using, as a dataset, second metabolic information and second growth index information for each of a plurality of second microbial colonies stored in a database, and genome analysis information on single strains constituting each of the plurality of second microbial colonies.

17. The computer device of claim 12,

wherein the processor
obtains second metabolic information and second growth index information related to each of a plurality of second microbial colonies stored in a database, and determines the strain combination by using the first metabolic information, the second metabolic information, the first growth index information, and the second growth index information as inputs of a second model.

18. The computer device of claim 17,

wherein the second model comprises a latent factor collaborative filtering algorithm model, and
wherein the processor
uses the target microorganism and a single strain as a user, uses third metabolic information and third growth index information of data concatenating the first metabolic information, the second metabolic information, the first growth index information, and the second growth index information as items, and determines the microbial strain combination for at least one of the third metabolic information and the third growth index information.

19. The computer device of claim 18,

wherein the processor
re-determines by determining weights for each of the estimated third metabolic information and third growth index information.

20. The computer device of claim 17,

wherein the second model comprises a transformer model, and
wherein the processor
learns a relationship between the target microorganism and a singlestrain, based on the first metabolic information, the second metabolic information, the first growth index information, and the second growth index information, and determines the strain combination, based on the relationship between the target microorganism and the single strain.

# FIG. 1

EP 4 679 432 A1

## FIG. 2

# FIG. 3

```
                        ┌─────────┐
                        │  START  │
                        └─────────┘
                             │
                             ▼
      ┌──────────────────────────────────────────────┐
      │     OBTAINING GENOME ANALYSIS INFORMATION     │
      │        RELATED TO TARGET MICROORGANISM        │──── S210
      └──────────────────────────────────────────────┘
                             │
                             ▼
      ┌──────────────────────────────────────────────┐
      │  OBTAINING FIRST METABOLIC INFORMATION RELATED│
      │        TO EACH OF A PLURALITY OF FIRST        │──── S220
      │               MICROBIAL COLONIES              │
      └──────────────────────────────────────────────┘
                             │
                             ▼
      ┌──────────────────────────────────────────────┐
      │    ESTIMATING FIRST GROWTH INDEX INFORMATION  │
      │   RELATED TO EACH OF THE PLURALITY OF FIRST   │
      │    MICROBIAL COLONIES BY USING THE GENOME     │──── S230
      │   ANALYSIS INFORMATION AND THE METABOLIC      │
      │     INFORMATION AS INPUTS TO A FIRST MODEL    │
      └──────────────────────────────────────────────┘
                             │
                             ▼
                        ┌─────────┐
                        │   END   │
                        └─────────┘
```

# FIG. 4

SHOTGUN ANALYSIS
PIPELINE INPUT

310
TARGET STRAIN
GENOME SEQUENCE

320
SHOTGUN ANALYSIS
PIPELINE

gene prediction

330
PREDICTED
GENE DATA

SHOTGUN ANALYSIS
PIPELINE OUTPUT

# FIG. 5

# FIG. 6

METABOLITE INTERACTIONS
WITHIN A MULTI STRAIN

mip      mro

# FIG. 7

510

DATA RELATED TO
A VIRTUAL MULTI
STRAIN SAMPLE

MIP

MRO

PREDICTED GENE DATA

mixed strain num

mixed strain list

PREDICTED MODEL
INPUT

530

MULTI STRAIN GROWTH
INDEX INFORMATION
PREDICTION MODEL

AI

ML

540

PREDICTED VALUES
FOR VIRTUAL MULTI
STRAIN SAMPLES

GROWTH INDEX
INFORMATION

PREDICTED MODEL
OUTPUT

mixed strain list

520

DATA RELATED TO
ACTUAL MULTI
STRAIN SAMPLES

MIP

MRO

PREDICTED GENE DATA

mixed strain num

mixed strain list

PREDICTED MODEL
TRAINING DATA

# FIG. 8

START

OBTAINING SECOND METABOLIC INFORMATION AND SECOND GROWTH INDEX INFORMATION RELATED TO EACH OF A PLURALITY OF SECOND MICROBIAL COLONIES STORED IN A DATABASE — S610

DETERMINE A STRAIN COMBINATION BY USING THE FIRST METABOLIC INFORMATION, THE SECOND METABOLIC INFORMATION, THE FIRST GROWTH INDEX INFORMATION, AND THE SECOND GROWTH INDEX INFORMATION AS INPUTS TO A SECOND MODEL — S620

END

## FIG. 9

# FIG. 10

810

LIST OF RECOMMENDED
STRAINS WITH GROWTH
INDEX INFORMATION

820

RANKING MODEL

830

MIP RECOMMENDED
STRAINS LIST

WEIGHT ASSIGNING
SYSTEM

FINAL RECOMMENDED
STRAINS LIST

RANKING MODEL OUTPUT

MRO RECOMMENDED
STRAINS LIST

RANKING MODEL INPUT

# FIG. 11

900

910

MEMORY

910

PROCESSOR

## INTERNATIONAL SEARCH REPORT

| International application No. |
| --- |
| **PCT/KR2024/002974** |

| | |
| --- | --- |
| **A. CLASSIFICATION OF SUBJECT MATTER** | |
| **G16B 40/20**(2019.01)i; **G16B 50/00**(2019.01)i; **C12N 1/20**(2006.01)i; **G06N 20/00**(2019.01)i | |
| According to International Patent Classification (IPC) or to both national classification and IPC | |

**B. FIELDS SEARCHED**

Minimum documentation searched (classification system followed by classification symbols)

G16B 40/20(2019.01); A61K 35/741(2015.01); C12Q 1/18(2006.01); G16B 5/00(2019.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 미생물 균주 조합(microbial strain combination), 게놈 분석 정보(genome analysis information), 대사 정보(metabolic information), 생장 지표 정보(growth indicator information), 모델(model), 대사 지원 중첩 (metabolic resource overlap), 대사 상호작용 잠재력(metabolic interaction potential)

**C. DOCUMENTS CONSIDERED TO BE RELEVANT**

| Category* | Citation of document, with indication, where appropriate, of the relevant passages | Relevant to claim No. |
| --- | --- | --- |
| Y | US 2020-0027524 A1 (GUSTO GLOBAL, LLC) 23 January 2020 (2020-01-23)<br>See abstract; claim 1; and paragraphs [0086], [0205], [0212] and [0215]. | 1-9,12-20 |
| A | | 10-11 |
| X | GOYAL, A. et al. Ecology-guided prediction of cross-feeding interactions in the human gut microbiome. Nature Communications. (electronic publication) 26 February 2021, vol. 12, thesis no. 1335, pp. 1-10.<br>See abstract; page 2, left column and page 4, right column, second paragraph; and figure 10. | 10-11 |
| Y | | 1-9,12-20 |
| A | SUN, X. et al. Keystone species determine the productivity of synthetic microbial biofilm communities. bioRxiv. 12 February 2022, version 2, lines 1-530.<br>See entire document. | 1-20 |
| A | COLARUSSO, A. V. et al. Computational modeling of metabolism in microbial communities on a genome-scale. Current Opinion in Systems Biology. 2021, vol. 26, pp. 46-57.<br>See entire document. | 1-20 |

| ✓ Further documents are listed in the continuation of Box C. | ✓ See patent family annex. |
| --- | --- |

| | | | |
| --- | --- | --- | --- |
| \* | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **11 June 2024** | **12 June 2024** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.

**PCT/KR2024/002974**

## C.    DOCUMENTS CONSIDERED TO BE RELEVANT

| Category* | Citation of document, with indication, where appropriate, of the relevant passages | Relevant to claim No. |
|---|---|---|
| A | MURRIETA-DUEÑAS, R. et al. Prediction of microbial growth via the hyperconic neural network approach. Chemical Engineering Research and Design. (electronic publication) 17 August 2022, vol. 186, pp. 525-540.<br>    See entire document. | 1-20 |
| A | WO 2023-006967 A1 (BACTEROMIC SP. Z O.O.) 02 February 2023 (2023-02-02)<br>    See entire document. | 1-20 |

Form PCT/ISA/210 (second sheet) (July 2022)

**INTERNATIONAL SEARCH REPORT**
**Information on patent family members**

International application No.

**PCT/KR2024/002974**

| Patent document cited in search report | | | Publication date (day/month/year) | Patent family member(s) | | | Publication date (day/month/year) |
|---|---|---|---|---|---|---|---|
| US | 2020-0027524 | A1 | 23 January 2020 | CA | 3058943 | A1 | 11 October 2018 |
| | | | | CA | 3058943 | C | 17 October 2023 |
| | | | | CA | 3211208 | A1 | 11 October 2018 |
| | | | | EP | 3606325 | A1 | 12 February 2020 |
| | | | | EP | 3606325 | A4 | 20 January 2021 |
| | | | | US | 11810650 | B2 | 07 November 2023 |
| | | | | US | 2024-0096440 | A1 | 21 March 2024 |
| | | | | WO | 2018-187272 | A1 | 11 October 2018 |
| WO | 2023-006967 | A1 | 02 February 2023 | CN | 117730351 | A | 19 March 2024 |
| | | | | EP | 4125064 | A1 | 01 February 2023 |

Form PCT/ISA/210 (patent family annex) (July 2022)

**REFERENCES CITED IN THE DESCRIPTION**

**Non-patent literature cited in the description**

- Metabolic dependencies drive species co-occurrence in diverse microbial communities. *PNAS*, 19 May 2015, vol. 112 (20), 6449-6454